# EUROPEAN PATENT APPLICATION

(11) **EP 4 699 520 A1**
(43) Date of publication of application: **25.02.2026**
(21) Application number: 24814593.0
(22) Date of filing: 31.05.2024
(51) Int. Cl.: A61B 5/00

(54) **METHOD AND DEVICE FOR MEASURING VISCOELASTICITY OF SURFACE OF BIOLOGICAL TISSUE**

(30) Priority: 02.06.2023 CN 202310646815
(71) Applicant: Beijing Tashan Technology Co., Ltd., Beijing 102308 (CN)
(72) Inventor: SUN, Tengchen, Beijing 102308 (CN); ZENG, Fanyou, Beijing 102308 (CN); WANG, Zhen, Beijing 102308 (CN)
(74) Representative: Yang, Shu
(86) International application number: PCT/CN2024/096666
(87) International publication number: WO 2024/245399

(57) **Abstract**

The present invention relates to a method and device for measuring the viscoelasticity of a biological tissue surface. The method comprises the following steps: S1 , in the normal direction of the biological tissue surface, driving the surface to move to a fixed displacement and then maintaining the displacement; S2 , using a sensor to measure the surface of the biological tissue during the implementation of step S1.

5. Time-varying mechanical response; S3. Based on the mechanical response acquired by the sensor, the processing module outputs viscoelastic characteristic data of the biological tissue surface. The measurement method of the present invention is intended to more scientifically and reliably detect the surface viscoelasticity of biological tissue, especially human skin.

## Description

### Technical Field

The invention relates to a method and a device for measuring the surface viscoelasticity of biological tissue, especially human skin.

### Background Art

Analyzing the physical properties of biological tissues, such as human skin or the epidermis of plant fruits, is crucial. For example, measuring the surface elasticity of human skin is valuable for both cosmetic and dermatological applications.

CK device currently available on the market uses technology such as that described in patent US5054502A , which utilizes negative pressure combined with optical measurement. During each test, the front end of the probe module's outer wall is pressed against the skin, causing deformation / displacement through vacuum suction or piston pressure . Measurement is then performed by reducing the intensity of light transmitted from the light transmitter to the light receiver. In actual products, such as the official website www.courage-khazaka.de The elasticity measurement products shown above utilize a vacuum-based negative pressure method . According to the measurement principle described on the CK website and technical manual, the CK device applies a fixed suction force to the skin for 0.1 seconds , observing changes and recovery before and after that 0.1 second. These parameters, such as R-parameters, F-parameters, and Q-parameters, are then used to evaluate the skin's viscoelastic properties. The CK method can be understood as observing the skin's stretching distance and subsequent recovery over a 0.1 second period using a fixed suction force. However, the empirical value for this 0.1 second time period is subject to considerable industry debate. For example, US scientists have suggested a range of 0.04s-0.07s as appropriate. Others suggest that this value should be individualized and will vary across test subjects. Currently, recognized limitations of the CK device include difficulties in data reproducibility and interpretation, as well as the lack of resolution caused by the negative pressure suction.

Elastic modulus and Elasticity of the Masticatory and FacialExpression Muscles in Patients with the Masticatory MusclePain≫ Korean J Oral Med , Vol. 34, No. 3, 2009 , the elasticity of human skin is about 0.70±0.46N .

The skin generates pressure. Since the skin has an elastic modulus and the skin in different parts of the body is interconnected, the squeezing of the skin covered by the front face (the squeezing force here is called external pressure, which refers to the pressure between the front face and the skin) will cause the elasticity of the skin in the hole of the front face to change. The degree of change is related to the degree of squeezing. If the external pressure used in each measurement is different, the pressure environment of each measurement will change, and the benchmark of each measurement will be different, which will cause interference to the measurement.

### Summary of the Invention

The present invention aims to propose a new measurement method for detecting the surface viscoelasticity of biological tissues, especially human skin, in a more scientific and reliable manner.

To this end, a method for measuring the viscoelasticity of the surface of biological tissue is proposed, comprising the following steps: S1. In the normal direction of the surface of the biological tissue, the surface is driven to move to a fixed displacement and then maintained; S2. A sensor is used to measure the time-varying mechanical response of the surface of the biological tissue during the implementation of step S1; S3. Based on the mechanical response obtained by the sensor, a processing module is used to output the viscoelastic characteristic data of the surface of the biological tissue.

The measurement method of the present invention does not employ the CK concept of measuring deformation with a fixed force. Instead, it measures the time-varying mechanical response of a biological tissue surface, such as human skin, by moving it a fixed distance (fixed-distance force measurement). Based on the mechanical response curve, relevant features (such as the various features mentioned above) are derived and provided to experts for evaluating the viscoelasticity of the tissue surface. Individual skin composition varies, and the mechanical response reflected by a fixed displacement of the skin varies. The present invention reflects these differences in the mechanical response through fixed-displacement force measurement, eliminating the need for empirical judgment. This results in more consistent and accurate measurement results for different test subjects, and theoretically, a more scientific measurement method.

The measuring method of the present invention is particularly suitable for measuring the viscoelasticity of human skin, and is also applicable to measuring the surface viscoelasticity of other biological tissues such as plant fruits.

When measuring the viscoelasticity of human skin, as an improved solution, in step S1, an actuator is configured to drive the sensor to apply an action to the surface of the biological tissue until the surface moves to a fixed displacement and then maintains the position, wherein the action at least includes mechanical pressing or mechanical stretching. For example, the CK device relies on negative pressure to stretch the skin, and a closed environment must be provided during the implementation, so the front end must be pressed against the skin.

The present invention provides the possibility of detecting the skin in a natural state that is not or substantially not damaged by using a sensor to perform mechanical action.

Skin, as a composite model of an elastic element obeying Hooke's law and a viscous element obeying Newton's law of viscosity , imposes certain requirements on the duration of mechanical motion. For example, when pressing, we want to minimize the diffusion of the skin caused by viscosity during compression, allowing for maximum release after compression for more accurate results. Therefore, pressing too slowly is unfeasible, while pressing too quickly also presents challenges. For example, accurate braking can lead to significant deviations in skin displacement. To balance detection accuracy, diffusion during compression , and displacement control, extensive experimentation has determined that the time from the sensor applying the motion to the surface moving normal to a fixed displacement in step S1 must be controlled within 0.1-1 seconds.

As another improvement to measuring skin viscoelasticity, the present method also includes step S4, which cyclically repeats steps S1-S3, controlling the fixed displacement of the surface along the normal direction to vary during each cycle to measure the mechanical responses of different skin layers. For example, during the first iteration of steps S1-S3 , the skin displacement is controlled between 0 and 2 mm to measure the mechanical response of the epidermis to compression or stretching . During the second iteration of steps S1-S3 , the skin displacement is controlled between 2 and 4.4 mm to measure the combined mechanical response of the epidermis and dermis, and similarly for the optional subcutaneous tissue. By controlling the displacement distance each time, the characteristics of different skin layers can be reflected. Furthermore, to prevent interference from the previous compression on the next measurement, a recovery period is provided between the end of each cycle and the beginning of the next cycle to allow the skin to return to its natural state.

As another improvement, the step S2 further includes: forming a mechanical curve based on the mechanical response obtained by the sensor ; the starting point of the mechanical curve is configured to be at least located at or before the point corresponding to the maximum value , and the maximum value is the value when the force of the surface feedback reaches the maximum; the ending point of the mechanical curve is configured to be at least located at or after the point corresponding to the steady-state value, and the steady-state value is the value when the force gradually decreases to a stable value after the maximum value, and the curve segment from the maximum value to the steady-state value can fully reflect the viscoelastic characteristics of the surface of the biological tissue. Wherein, the method for judging the steady-state value further includes: taking the value corresponding to the first point on the mechanical curve after the maximum value whose slope is less than the set threshold as the steady-state value; or taking the value corresponding to the set time after the maximum value on the mechanical curve.

Preferably, a prompting device can be provided to prompt when the sensor obtains the end point, for example, through sound/light/vibration.

The present invention also provides a device for measuring the viscoelasticity of the surface of biological tissue, comprising: a sensor for measuring the mechanical response of the surface when an action is applied to the surface of the biological tissue; an actuator for driving the sensor to perform the action; a processing module respectively coupled to the actuator and the sensor; and a memory arranged to store computer-executable instructions, which, when executed, causes the processing module to execute the above-mentioned method.

To prevent interference with surface structure elasticity measurements caused by compression from the front end, which disrupts the surface structure's natural state, an improved solution includes a measuring device with a distance detection device and a reference surface facing the surface during measurement. The sensor is configured to contact the surface protruding from the reference surface during measurement, thereby achieving non-contact between the reference surface and the surface. The distance detection device is used to obtain the distance between the reference surface and the biological tissue surface. During measurement, the distance between the reference surface and the measured surface structure is determined by the distance detection device. An external system, such as a robotic arm, moves the module to a position where the distance from the reference surface to the surface structure remains consistent, ensuring a stable measurement reference and environment. The sensor then contacts the surface structure protruding from the reference surface during measurement. Since the reference surface and the surface structure are non-contact, compression is avoided, eliminating pressure interference at its source . This allows the sensor to apply compression or tension to the surface in its natural state (undamaged by compression) and measure the mechanical response, resulting in more accurate measurement results. In this solution, the reference surface should be understood as the outer surface of the module housing that bears the sensor.

Furthermore, the distance detection device is configured to include at least two distance sensing electrodes provided on the reference plane, wherein the distance sensing electrodes are provided on the front or back of the reference plane; the contactless measurement module is provided with a capacitance-to-digital conversion circuit (CDC) of a coupling processing module, and the capacitance-to-digital conversion circuit couples each distance sensing electrode to obtain mutual capacitance; the processing module outputs distance information based on the mutual capacitance . Specifically, the mutual capacitance value formed by the distance sensing electrode changes when it approaches a surface structure such as human skin, thereby obtaining the distance between the human skin and the reference plane, controlling the distance between the human skin and the reference plane to reach a preset value and ensuring that it remains stable. In the above scheme, based on a pair of electrodes, only

In order to obtain more accurate distance information, the number of distance detection devices can be configured to be at least two, and they can be arranged around the sensor. The area and/ or spacing of the distance sensing electrodes in each distance detection device are different, so that the strength of the electric field lines formed is different, that is, the electric field lines formed are high and low. On the one hand, the measurement range is expanded to serve as a reference for the operating speed of the external actuator. More importantly, the height difference formed by the electric field lines can be used to obtain the accurate degree of proximity.

Alternatively, as another improved solution, the reference surface can be controlled to be in contact with the skin during each surface structure measurement . Specifically, the measuring device is configured to include a pressure detection device and a reference surface that directly or indirectly contacts the surface during measurement; the pressure detection device is used to sense the contact pressure between the reference surface and the surface when the reference surface directly or indirectly contacts the surface. During the measurement process, the pressure detection device is used to feedback the contact pressure (external pressure) between the reference surface and the surface structure, such as human skin. An external system, such as a manipulator, moves the module to adjust the contact pressure to be consistent with the previous measurement, for example, a contact force of 0.5N . On the one hand, there is substantially no compression, thereby substantially not destroying the natural state of the skin. On the other hand, the sensor maintains a uniform external pressure measurement environment in each measurement, avoiding the introduction of measurement errors. This allows the sensor to apply a pressing or stretching action under a stable measurement reference to measure the mechanical response of the surface structure, resulting in more accurate measurement results. Similarly, the number of pressure detection devices is configured to be at least two, and they are arranged around the sensor to take into account all circumferential positions and ensure detection uniformity. Preferably, the number of pressure detection devices is set to at least three and they are evenly distributed around the sensor.

For the scheme of using a sensor to press a surface structure, the sensor can be configured to include a pressure detection sensor, which is coupled to an actuator, wherein the actuator is used to drive the pressure detection sensor to move along the normal direction of contact with the surface structure during measurement. Furthermore, the pressure detection sensor is configured as a first sensor unit, which is provided with a flexible multifunctional layer, wherein a curved elastic electrode electrically connected to the multifunctional layer is provided inside the flexible multifunctional layer as an upper electrode, a lower electrode is provided below the upper electrode, an insulating layer is provided between the upper electrode and the lower electrode, and the downward projection of the upper electrode covers at least part of the area of the lower electrode, and the deformation of the flexible multifunctional layer by an external force drives the upper electrode to change the contact area with the insulating layer; the non-contact measurement module is provided with a capacitance-to-digital conversion circuit (CDC) of a coupling processing module, and the capacitance-to-digital conversion circuit couples each electrode through a switch array.

This sensor measures the capacitance formed between the upper and lower electrodes in the first sensor unit. When pressed, the deformation of the flexible multifunctional layer and the capacitance formed between the upper and lower electrodes reflect the normal component of the force . Combined with CDC to collect capacitance values from each electrode, this allows for high force resolution. Furthermore, due to the direct tactile measurement, elastic force can be accurately measured.

As for the scheme of using sensors for stretching, the sensor can be configured to include a tension detection sensor, wherein the surface of the tension detection sensor for contacting the surface structure is provided with an adhesive layer, such as double-sided tape; the tension detection sensor is coupled to an actuator, and the actuator is used to drive the tension detection sensor to move along the normal direction of contact with the surface structure during measurement. During detection, when the tension detection sensor contacts the surface structure such as human skin, the surface double-sided tape adheres to the human skin, providing a pre-adhesive force, and the actuator drives the tension detection sensor to move. The tension detection sensor is in a stretched state and generates a tensile force. The tensile force and the pre-adhesive force can be used to derive the actual contact stress between the human skin and the sensor. In the present utility model, after the detection is completed, a protective cover can be installed to prevent the double-sided tape from being in long-term contact with the air to cause an oxidation reaction and change in viscosity; alternatively, the double-sided tape can be made into a consumable material, and when testing is required, the double-sided tape is placed in the groove of the watch case, and the double-sided tape is removed after monitoring is completed, and the bonding position is wiped to ensure that the pre-adhesive force of each test remains relatively stable. Illustrations
FIG1 shows a schematic flow chart of a method for measuring viscoelasticity of biological tissue surfaces;
Figure 2 shows the relative position relationship between the biological tissue surface viscoelasticity measurement device and the tissue surface picture;
FIG3 shows a schematic structural diagram of a device for measuring viscoelasticity of a biological tissue surface;
FIG4 is a schematic diagram showing the effect of the measurement device when the sensor is pressed to the depth of the epidermis;
FIG5 is a schematic diagram showing the effect of the measurement device when the sensor is pressed to the depth of the dermis;
FIG6 shows a schematic diagram of the structural distribution of the distance detection device;
Figure 7-1 shows the electric field line distribution structure of the distance detection electrode located on the skin side of the reference surface.
Figure 7-2 shows the electric field line distribution structure of the distance detection electrode located on the side of the reference surface facing away from the skin.
FIG8 shows a schematic structural diagram of a pressing scheme of a test device for viscoelasticity of biological tissue surface;
FIG9 shows a schematic diagram of the distribution relationship between the flexible upper electrode and the lower electrode;
Figure 10-1 shows a schematic diagram of the grouping and distribution relationship between the flexible multifunctional layer and the lower layer electrodes;
Figure 10-2 shows a schematic diagram of the relationship between the grouping of the flexible multifunctional layer and the distribution of the lower layer electrodes;
FIG11 shows a schematic structural diagram of a stretching scheme of a test device for viscoelasticity of a biological tissue surface;
Figure 12 shows a schematic diagram of the protective cover installation structure after the detection is completed .

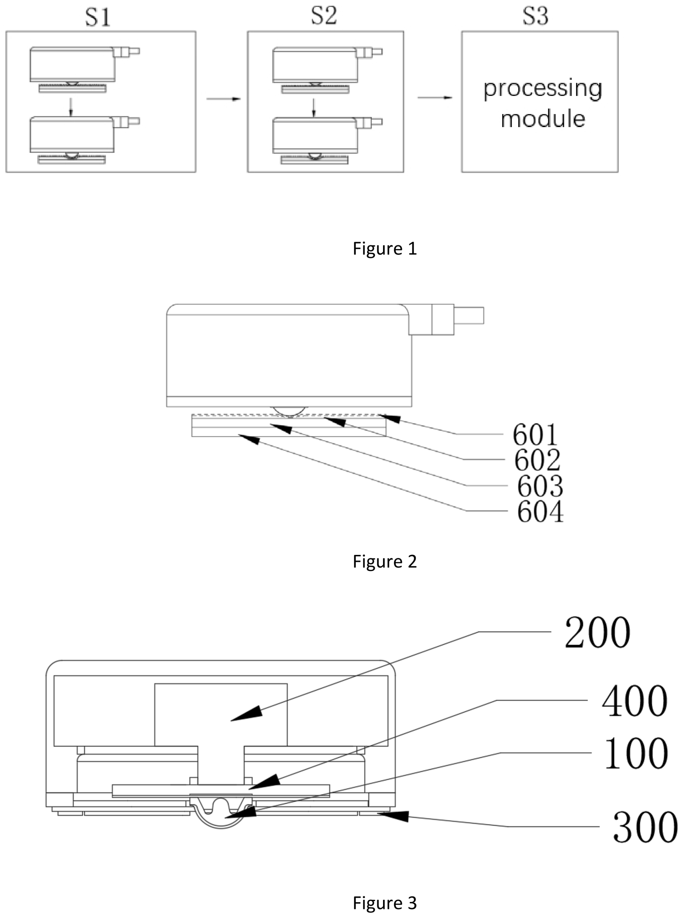

Figure 1, a flow chart of a method for measuring the viscoelasticity of a biological tissue surface mainly includes three steps: S1, driving a measuring device to move along the normal direction of the biological tissue surface to a fixed displacement and then maintaining it; S2 , synchronously with S1, using a sensor to measure the time-varying mechanical response of the biological tissue surface during the implementation of step S1 ; S3 , using a processing module to output viscoelastic characteristic data of the biological tissue surface based on the mechanical response acquired by the sensor.

In FIG2, the surface tissue measured by the biological tissue surface viscoelasticity measuring device is configured as skin, which is mainly composed of a stratum corneum 601, an epidermis 602, a dermis 603, and a subcutaneous tissue 604.

Figure 3, the biological tissue surface viscoelasticity measuring device mainly consists of a sensor 100, an actuator 200, a distance detection device 300, and a processing module 400. In step S1, the actuator 200 drives the sensor 100 to stretch or press the surface structure to reach a predetermined position, and the sensor 100 reflects the surface structure.
is analyzed by processing module 400 to determine the contact stress between sensor 100 and the skin surface, completing the mechanical response data required for step S2 . The time required for sensor 100 to reach the normal preset position from the start of movement in step S1 can be configured as a fixed time. The measurement device can be controlled by an external robotic arm to ensure that the measurement position is relatively fixed each time. During the test, feedback from the distance detection device 300 ensures that the relative position between the reference surface and the epidermal structure remains stable.

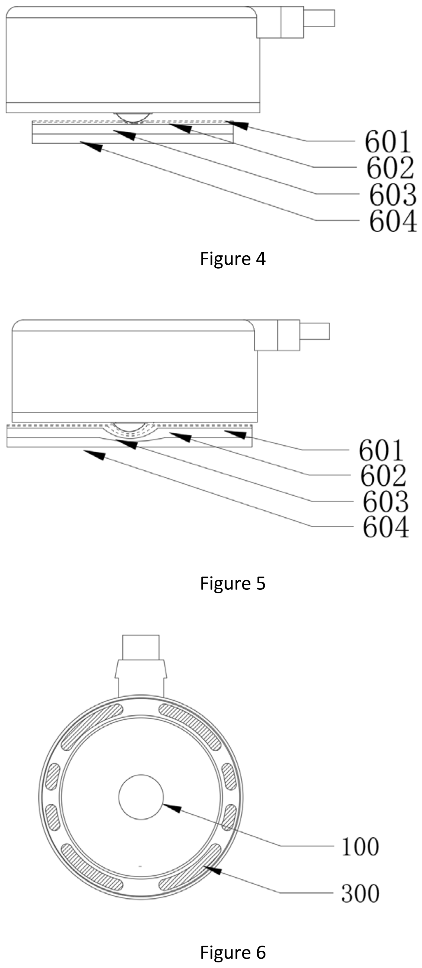

FIG4 and FIG5 , each measurement process of the measuring device needs to include steps S1-S3 and step S4. Step S4 is to execute steps S1-S3 in a loop . During the repeated cycle, the displacement of the sensor 100 driven by the actuator 200 may vary according to the instructions of the processing module 400 , depending on the skin surface.

The depth of the layer is determined by the preset displacement of different sensors 100, and the viscoelastic characteristics of the deep skin

The value can be calculated by removing the previous test data from the mechanical response curve measured at that depth to represent the viscoelastic characteristics of the skin components at that depth, thereby effectively detecting the viscoelastic properties of components at different depths. After each measurement, sufficient time must be allowed between cyclic measurements to allow the surface of the biological tissue, such as skin, to return to its natural state, avoiding inconsistencies in the initial state during repeated measurements. Driven by actuator 200 , sensor 100 stretches or compresses the epidermal tissue. The starting point of the mechanical response curve for the epidermal tissue stress condition is configured to be at least at or before the point corresponding to the maximum contact force, which is the value at which the force feedback from the surface tissue reaches its maximum. The ending point of the mechanical response curve is configured to be at least at or after the point corresponding to the steady-state value, which is the value at which the force gradually decreases to a stable value after the maximum value. The steady-state value can be determined by determining the steady-state value by taking the value corresponding to the first point on the mechanical curve after the maximum value where the slope of the mechanical change is less than a set threshold as the steady-state value, or by taking the value of the point on the mechanical curve that is a set time period after the maximum value as the steady-state value. When the force response curve reaches the end point, that is, when the epidermis returns to its initial state, the detection device can issue a prompt, which can be a voice prompt through the buzzer on the processing module, or an image prompt through the external display of the measuring device, or a light prompt through devices such as light-emitting diodes.

In FIG6, the distance detection device 300 mainly includes a distance detection electrode.

At least two sets of electrodes are included, and the sensor 100 protrudes from the distance detection device. When the test device approaches the epidermal tissue

When the skin is touched, the capacitance value formed by the distance detection electrode changes. The processing module 400 can determine the distance between the test device and the skin, thereby ensuring that the position of the test device and the human skin is relatively stable each time it is measured. Since the sensor 100 protrudes from the distance detection device, the distance detection device 300 will not come into direct contact with the skin , which can avoid skin damage and external force interference during the test process.

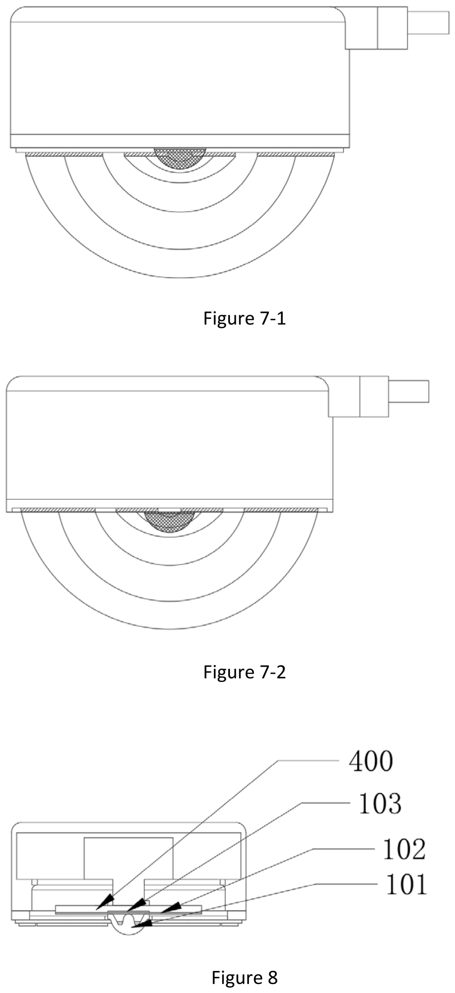

Figure 7-1 and Figure 7-2 , the distance detection electrodes can be distributed on the side of the reference surface close to the human skin or the side facing away from the human skin. The distance detection electrodes can be configured with at least two electrode groups. The distance electrodes can also be configured into electrode groups with different areas and spacings to form different electric field line distributions, thereby improving the overall detection distance range while subdividing the detection distance and accurately judging the relative position relationship between the reference surface and the human skin.

In FIG8, the device for measuring the viscoelasticity of the biological tissue surface is a sensor 100 in a pressing structure. The sensor 100 is mainly composed of a flexible upper electrode 101 ( composed of a flexible multifunctional layer at the bottom and an upward electrode located in the middle of the multifunctional layer).

The upper electrode protruding into a curved hemisphere is integrally formed), the insulating layer 102 is provided in the processing module 400

The lower electrode 103 is included in the processing module 400 and can also be separately provided.

The sensor 100 can move along the normal direction of the epidermal structure under the drive of the actuator 200 to achieve

The distance detection device 300 limits the initial state of the detection module and the epidermal structure.

The sensor 100 is pressed by the actuator 200, and due to the different viscoelasticity of the epidermal structure,

In the same displacement state, the deformation amount of the flexible multifunctional layer of the upper electrode 101 is different, and the processing module
400 determines the viscoelastic difference of the epidermal structure by the capacitance value change of the sensor 100.

The real-time detection of the capacitance change caused by the deformation of the upper electrode 101 of 100 can be used to obtain the test process.

The force response curve of the epidermal tissue is obtained by processing module 400 to obtain the viscoelastic characteristic parameters of the epidermal tissue.

Multiple sensors 100 can be provided to obtain multiple group averages to reduce measurement errors.

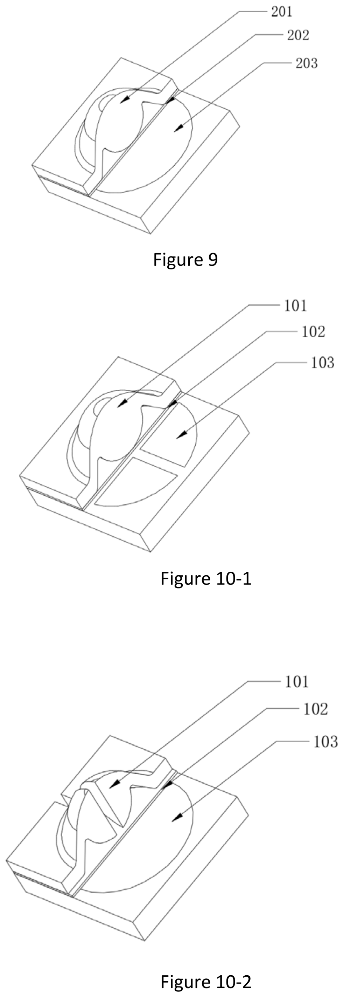

in FIG9 , the flexible upper electrode 101 is subjected to external force and the insulating layer on the surface of the lower electrode 103
102 contacts, and due to the pressure, the flexible upper electrode 101 is deformed, and the flexible upper electrode 101 and
the lower electrode 103 changes, and the magnitude of the normal force applied to the sensor 100 can be obtained through the change in capacitance value.

As shown in FIG10-1 , the lower electrode 103 can also be configured as a plurality of distributed electrodes, which can be configured as strips.
three detection electrodes are arranged in different directions according to deformation . When the flexible multifunctional layer of the upper electrode 101 is deformed by force, the projected area of the lower electrode partition changes differently according to the magnitude of the force direction, resulting in different capacitance value change information. The upper electrode 101 is switched by the processing module 400.

The array couples different combinations of upper and lower electrodes, and thus the actual force magnitude and direction information of the upper electrode 101 can be obtained according to the different contact areas.

As shown in FIG10-2, the upper electrode 101 can also be divided into multiple parts, each part is connected by

The insulating layers are bonded together, and there is no conduction between the parts. The lower electrode 103 is a whole and serves as a common electrode. The magnitude and direction information of the force can also be obtained according to the capacitance change values of different electrode components.

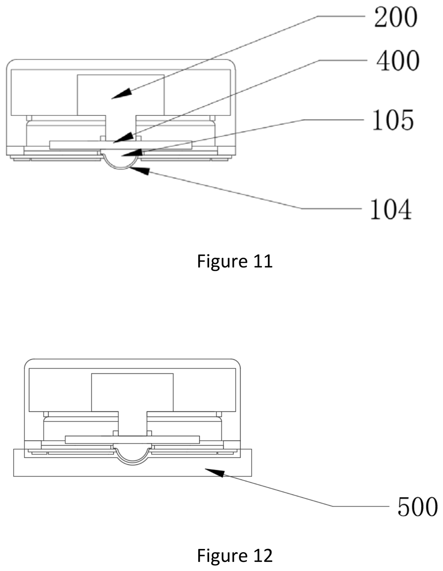

In FIG 11 , when the measuring device detects the surface viscoelasticity in a stretched state, the sensor 100 mainly
The tension detection sensor 105 is composed of an adhesive layer 104 , and the tension detection sensor 105 is fixed to the adhesive layer 104.

Actuator 200 drives the tension detection sensor 105 to generate relative force with the surface tissue during measurement.

Normal movement, the tension detection sensor 105 contacts the surface tissue such as human skin, and the adhesive layer 104 is like a double

The three adhesive layers are fixed together, and the adhesive layer 104 provides pre-adhesive force. The actuator 200 drives the tension detection sensor.

The sensor 105 moves in the normal direction, and the tension detection sensor 105 is in a stretched state to generate a stretching force. The stretching force and the pre-adhesion force can be used to derive the actual contact stress between the human skin and the sensor 105. Through steps S1-S2 , the mechanical response curve of the surface tissue under the tensile force can be obtained, and the viscoelastic characteristic parameters of the surface tissue are output through the processing module 400 .

In FIG12 , after the test is completed, the protective cover 500 can be installed to prevent the double-sided adhesive from being in long-term contact with the air and causing oxidation reaction to change the viscosity; alternatively, the adhesive layer 104 can be made into a consumable material, and when the test is required, the protective cover 500 can be installed.

The adhesive layer 104 is placed in the groove of the watch case. After the test is completed, the adhesive layer 104 is removed and the bonding position is wiped to ensure that the pre-bonding force of each test remains relatively stable.

Finally, it should be noted that the above embodiments are only used to illustrate the technical solutions of the present invention, rather than to limit the scope of protection of the present invention. Although the present invention has been described in detail with reference to the preferred embodiments, those skilled in the art should understand that the technical solutions of the present invention may be modified or replaced by equivalents without departing from the essence and scope of the technical solutions of the present invention.

## Claims

1. A method for measuring the viscoelasticity of a biological tissue surface, comprising the following steps:
S1. driving, in a normal direction to the surface of the biological tissue, the surface to move to a fixed displacement and then maintaining the displacement;
S2. using a sensor (100) to measure a time-varying mechanical response of the surface of the biological tissue during implementation of step S1;
S3. outputting viscoelastic characteristic data of the surface of the biological tissue by a processing module (400) based on the mechanical response acquired by the sensor (100).

2. The measurement method according to claim 1, wherein the biological tissue is configured as skin.

3. The measurement method according to claim 2, wherein step S1 further comprises:
using an actuator (200) to drive the sensor (100) to apply an action to the surface of the biological tissue until the surface moves to the fixed displacement and then maintains the displacement, wherein the action at least includes mechanical pressing or mechanical stretching.

4. The measurement method according to claim 3, wherein a time from when the sensor (100) starts to apply the action to when the surface moves along the normal direction to the fixed displacement in step S1 is configured to be within 0.1-1 s.

5. The measurement method according to claim 2, further comprising step S4 for cyclically repeating steps S1-S3, and controlling the fixed displacement of the surface moving along the normal direction to be different in each cycle so as to obtain mechanical responses of different layers of the skin.

6. The measurement method according to claim 5, wherein a recovery time is reserved after each cycle ends and before a next cycle begins for the skin to return to its natural state.

7. The measurement method according to claim 1, wherein step S2 further comprises:
forming a mechanical curve based on the mechanical response obtained by the "sensor (100)";
a starting point of the mechanical curve is configured to be at least located at or before a point corresponding to a maximum value, wherein the maximum value is a value when a force fed back by the surface reaches a maximum;
an end point of the mechanical curve is configured to be at least located at or after a point corresponding to a steady-state value, wherein the steady-state value is a value when the force gradually decreases to a stable value after the maximum value.

8. The measurement method according to claim 7, wherein the method for determining the steady-state value further comprises:
taking a value corresponding to a first point after the maximum value on the mechanical curve whose slope is less than a set threshold as the steady-state value; or,
taking a value of a point corresponding to a set time length after the maximum value on the mechanical curve as the steady-state value.

9. A device for measuring the viscoelasticity of a biological tissue surface, comprising:
a sensor (100) for measuring a mechanical response of the surface of the biological tissue when an action is applied to the surface of the biological tissue;
an actuator (200) for driving the sensor (100) to perform the action;
a processing module (400), coupled to the actuator (200) and the sensor (100) respectively;
a memory arranged to store computer executable instructions, which when executed cause the processing module (400) to perform the method according to any one of claims 1 to 8.

10. The measuring device according to claim 9, wherein:
the measuring device has a distance detection device (300) and a reference surface facing the surface during measurement;
the sensor (100) is configured to contact the surface protruding from the reference surface during measurement to achieve non-contact between the reference surface and the surface;
the distance detection device (300) is configured to obtain a distance between the reference surface and the surface of the biological tissue.

11. The measuring device according to claim 10, wherein:
the distance detection device (300) is configured to include at least two distance sensing electrodes provided on the reference surface;
the non-contact measurement module is provided with a capacitance-to-digital conversion circuit coupled to the processing module (400), and the capacitance-to-digital conversion circuit couples each distance sensing electrode to obtain a mutual capacitance;
the processing module (400) outputs distance information between the reference surface and the biological tissue surface according to the mutual capacitance.

12. The measuring device according to claim 11, wherein a number of the distance detection devices (300) is configured to be at least two and arranged around the sensor (100), and an area and/or spacing of the distance sensing electrodes in each distance detection device (300) is different.

13. The measuring device according to claim 9, wherein:
the measuring device has a pressure detection device and a reference surface that directly or indirectly bears against the surface during measurement;
the pressure detection device is configured to sense a contact pressure between the reference surface and the surface when the reference surface directly or indirectly presses against the surface.

14. The measuring device according to claim 9, wherein the sensor (100) is configured to include a pressure detection sensor;
the pressure detection sensor is coupled to the actuator (200), and the actuator (200) is configured to drive the pressure detection sensor to move along a normal direction of contact with the surface during measurement.

15. The measuring device according to claim 14, wherein:
the pressure detection sensor is configured as a first sensor unit, which includes a flexible multifunctional layer (101), a curved elastic electrode electrically connected to the multifunctional layer as an upper electrode (101) disposed within the flexible multifunctional layer (101), a lower electrode (103) disposed below the upper electrode (101), an insulating layer (102) disposed between the upper electrode (101) and the lower electrode (103), and a downward projection of the upper electrode (101) covering at least a portion of the lower electrode (103), and deformation of the flexible multifunctional layer (101) caused by an external force drives the upper electrode (101) to change a contact area with the insulating layer (102);
the non-contact measurement module is provided with a capacitance-to-digital conversion circuit coupled with the processing module (400), the capacitance-to-digital conversion circuit couples the electrodes via a switch array for a capacitance formed between the upper electrode (101) and the lower electrode (103) in the first sensor unit.

16. The measuring device according to claim 9, wherein the sensor (100) is configured to include a tension detection sensor (105), wherein a surface of the tension detection sensor (105) for contacting the surface is provided with an adhesive layer (104);
the tension detection sensor (105) is coupled to the actuator (200), and the actuator (200) is configured to drive the tension detection sensor (105) to move in a normal direction in contact with the surface during measurement.
